# EUROPEAN PATENT SPECIFICATION

(11) **EP 0 410 658 B1**
(45) Date of publication and mention of the grant of the patent: **27.09.1995**
(21) Application number: 90308020.8
(22) Date of filing: 23.07.1990
(51) Int. Cl.: A61B 5/0245, G01R 29/027

(54) **Pulsimeter**
Pulsmeter
Mesure de la pulsation

(30) Priority: 25.07.1989 JP 193499/89; 25.07.1989 JP 193501/89
(43) Date of publication of application: 30.01.1991
(73) Proprietor: SEIKO INSTRUMENTS INC., Tokyo 136 (JP)
(72) Inventor: Odagiri, Hirshi, c/o Seiko Instruments Inc., Tokyo (JP); Masaki, Hiryuki, c/o Seiko Instruments Inc., Tokyo (JP); Inoue, Yuichi, c/o Seiko Instruments Inc., Tokyo (JP)
(74) Representative: Sturt, Clifford Mark

(56) References cited:
- WO-A-89/01633
- DE-A- 2 327 430
- US-A- 3 593 162
- US-A- 4 775 840
- ELECTRONICS INTERNATIONAL vol. 54, no. 14, July 1981,pages 125-127, New York, US; T.G. BARNETT et al.: "Pulse-width monitor flags poor timing"
- J. MARKUS, "Modern Electronic Circuits Reference Manual", 1980 McGraw-Hill Inc., page 527

## Description

This invention relates to a pulsimeter, in particular to a photo-electric type pulsimeter.

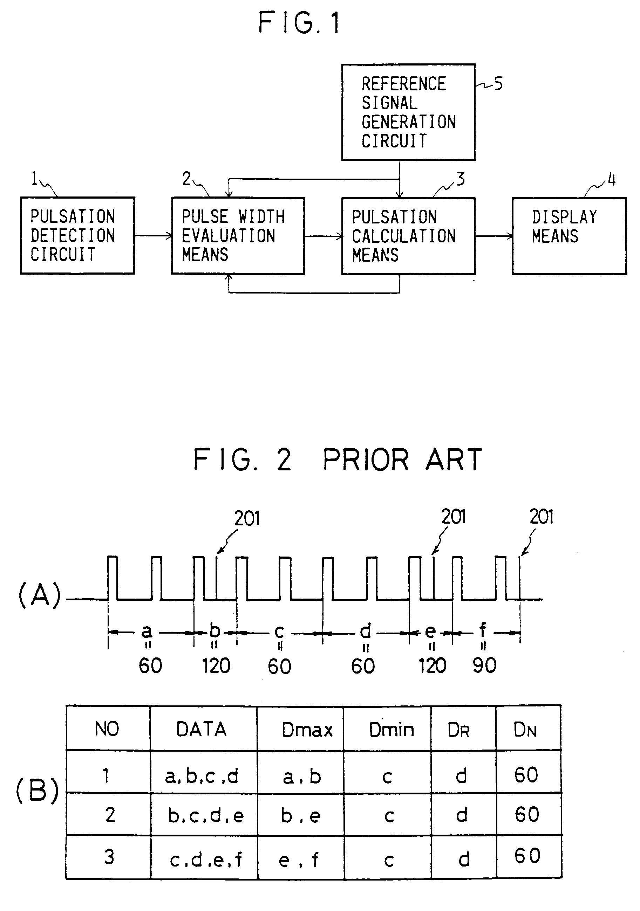

Various attempts have been made in the past in order to stabilise the pulsation display of a portable pulsimeter. Most of them relate to a method of processing values for the pulse rate per minute when this is calculated from the period of the output signal obtained from a pulsation detection circuit. As an example, a processing system called a "4-data selection movement system" will be explained. The 4-data selection movement system is a system which removes the two maximum values of four pulsation conversion data values Dₘₐₓ and one minimum value Dₘᵢₙ, and then displays the one remaining data value D_{R} or D_{N}. A specific example is shown in Figure 2. Figure 2 (A) shows the output of the pulsation detection circuit where noise 201 arises intermittently. Symbols a, b, c, d, e, and f, in Figure 2 represent values for the pulse rate per minute converted from the average of the periods of two successive pulses. It can be understood from Figure 2 (B) that the display value D_{N} is stable in spite of the intermittent noise 201.

A photoelectric pulsation type pulsimeter according to the preamble portion of present independent claim 1 is known from document J. MARKUS, "Modern Electronic Circuits Reference Manual", 1980 McGraw-Hill Inc. page 527.

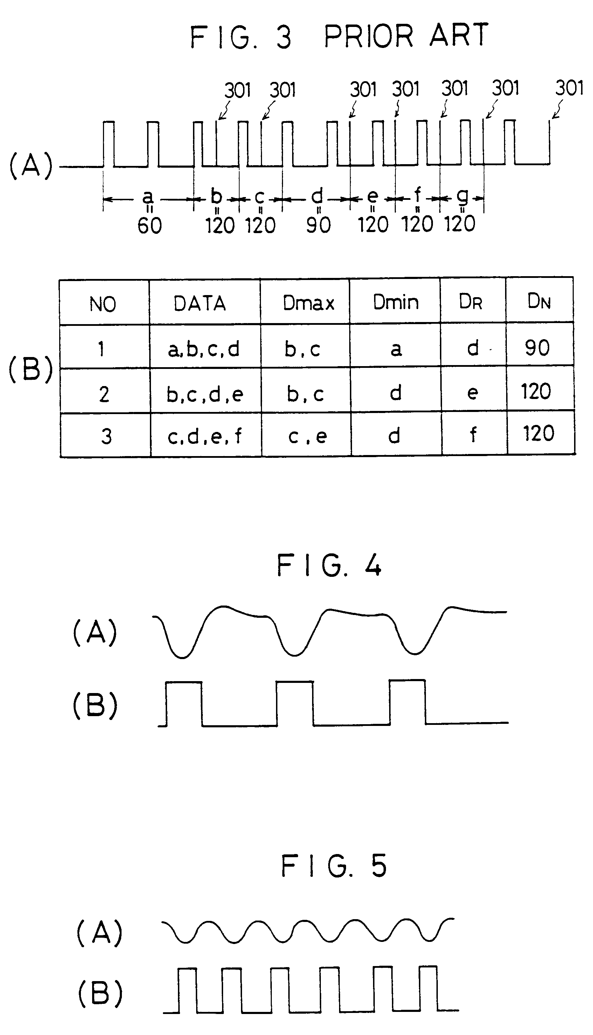

As described above, the prior art technique is effective for intermittent noise, but it is not effective for continuous noise. Figure 3 (A) shows the output when continuous noise 301 exists. The output of the pulsation detection circuit shown in Figure 3 (A) is exactly the same as that of Figure 2 (A) described above, except for the noise 301. In accordance with the above method which aims to eliminate noise by data processing, however, the noise 2 is recognised as the pulsation whose rate is to be calculated, and it too is processed, so that there is a limit to stability and accuracy of the pulse rate display.

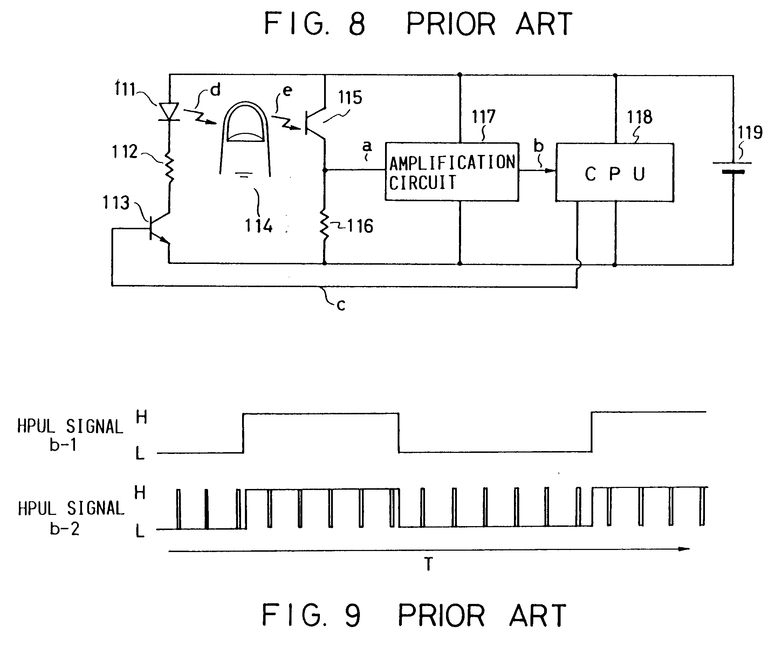

On the other hand, an LED or the like has been used conventionally as a light emission device of a pulsimeter for detecting the change of blood flow rate by the use of optical means and for counting and displaying a pulse rate. If this LED is turned on continuously during measurement, the consumed current becomes extremely great. Therefore, an attempt has been made to save the consumed current by turning on the LED for a minimum light emission time in which a change of the blood flow rate can be grasped as an electric signal by means of a light emission device. The operation of this prior art device will be explained briefly with reference to a schematic structural view of a photoelectric pulsimeter shown in Figure 8.

An LED 111 is used as the light emission device, which emits light continuously when a light emission signal c is applied from a CPU 118 to a switching transistor 113. The intensity of light is regulated by a current limiting resistor 112. The emitted infra-red rays impinge on a finger 114 of a subject, and the blood flow rate is represented by the intensity of light transmitted to a light reception device 115. A photo-transistor is used as this light reception device. Since the photo-transistor 115 generates a current in accordance with the intensity of the received light, a resistor 116 generates a blood flow voltage a in accordance with the blood flow rate in the finger. This blood flow voltage a is amplified and shaped by an amplification circuit 117 and output as a HPUL signal b which is in synchronism with the pulsation. The HPUL signal b is input to the CPU 118, which calculates the pulse rate per unit time and the like from the input period of the HPUL signal and writes the data into a memory, not shown, or displays it. Reference numeral 119 represents a battery for supplying current in the circuit described above.

If a battery is used as the power source of the pulsimeter described above, the internal resistance of the battery becomes high when the battery capacity drops or when the ambient temperature is low. In such a case, the power source voltage drops due to a voltage drop caused by the current flowing through the light emission device when it is turned on, and a spike pulse which is in synchronism with turn on of the light emission device occurs in a waveform shaping circuit providing the final output stage of the amplification circuit and is like to overlap with the HPUL signal.

Figure 9 shows the condition described above. Under the normal state, the HPUL signal b is only the pulse signal which is in synchronism with the pulsation as shown in b-1. But if the spike pulse occurs for the reason described, it overlaps with the HPUL signal as shown on b-2 and if the CPU 119 recognises this spike pulse as the HPUL signal, it cannot calculate the pulse rate of the pulsation correctly.

It is an object of the present invention to overcome the problems described above.

According to the present invention, there is provided a photoelectric pulsation type pulsimeter comprising a pulsation detection circuit, which includes a photoelectric element for detecting blood flow rate and for generating a corresponding pulse signal, pulsation calculation means for calculating a pulse rate in response to the pulse signal, reference signal generating means for generating a reference signal and for outputting the reference signal to the pulsation calculation means, characterised in that the pulsimeter includes pulse width evaluation means to which the reference signal is outputted, in that the pulse width evaluation means evaluates the pulse signal output from the pulsation detection circuit and in that the pulse width evaluation means are arranged to change the evaluation reference according to a pulse rate previously calculated by the pulsation calculation means.

The present invention interposes pulse width evaluation means for evaluating the pulse width of the signal output from a pulsation detection circuit between this pulsation detection circuit and pulsation calculation means, on the basis that a pulse resulting from noise will have a relatively small pulse width.

The pulse width evaluation means evaluates the pulse signal output from the pulsation detection circuit and transmits only the signal which it recognises as the normal pulse signal to the pulsation calculation means. In this manner, noise input continuously can be eliminated and stability of a pulsation display can be improved drastically.

In a preferred embodiment of the present invention described below, an HPUL signal generated in response to light emission by a light emission device is masked in a specific period after turn on of the light emission device so as to prevent erroneous recognition of a spike pulse as the correct HPUL signal. A CPU in this embodiment employed for calculating pulse rate thus does not erroneously recognise the spike pulse as the HPUL signal but can recognise only the correct HPUL signal which is in synchronism with the pulsation and can, therefore, calculate the correct pulse rate.

The invention is described further, by way of example, with reference to the accompanying drawings, in which:-
Figure 1 is a block diagram showing the basic structure of a first embodiment of pulsimeter according to the present invention;
Figure 2 is a diagram for illustrating the operation of a 4-data selection movement system when noise occurs intermittently;
Figure 3 is a diagram for illustrating the operation of the 4-data selection movement system when noise occurs continuously;
Figure 4 is a diagram showing the signals in a pulsation detection circuit when a pulse rate is low;
Figure 5 is a diagram showing the signals in the pulsation detection circuit when the pulse rate is high;
Figure 6 is a circuit diagram showing a first version of the pulsimeter of Figure 1;
Figure 7 is a flow chart representing a second version of the pulsimeter of Figure 1;
Figure 8 is a diagram of a prior art pulsimeter;
Figure 9 is a diagram illustrating signals arising in the pulsimeter of Figure 8;
Figure 10 is a flow chart representing the pulsimeter of Figure 8 adapted according to the present invention;
Figure 11 is a circuit diagram of parts of the pulsimeter of Figure 8 adapted according to the present invention; and
Figure 12 is a timing chart for signals arising in the circuitry of Figure 11.

Figure 1 is a block diagram showing the basic structure of an embodiment of the present invention. A pulsation signal detected by a pulsation detection circuit 1 is output as a pulse signal to pulse width evaluation means 2. The pulse width evaluation means 2 evaluates the pulse signal output from the pulsation detection circuit 1 on the basis of a pulse rate previously determined by pulsation calculation means 3 and an evaluation reference determined from a reference signal generation circuit 5. If the pulse signal is found to have a pulse width above a reference pulse width as a result of the evaluation, the pulse width evaluation means 2 transmits the received pulse signal to the pulsation calculation means 3. The pulsation calculation means 3 calculates a pulse rate per minute from the period of the pulse signal transmitted from the pulse width evaluation means 2 and outputs the result to display means 4.

Since the pulse width evaluation means 2 is disposed as described above, those pulses which do not have a pulse width exceeding a certain reference pulse width are not recognised as the pulsation signal, and the pulse rate displayed by the display means 4 becomes stabilised.

A plurality of the evaluation references are prepared for the pulse width evaluation means 2 depending on the content of the pulsation calculation means 3 for reasons which will now be explained. If the pulsation detection circuit 1 consists of an amplification circuit, a filter and a simple waveform shaping circuit in the case of photo-electric pulsation detection, the pulse width of the pulse signal changes in accordance with the pulse rate as shown in Figure 4 and Figure 5. Figure 4 (A) and Figure 5 (A) show the analog waveform of the pulsation signal, and Figure 4 (B) and Figure 5 (B) show the output waveform of the corresponding pulse signal. The pulse width is small when the pulse rate is high and is large when the latter is low. Therefore, if there is only one evaluation reference for the pulse width evaluation means 2, it must be set to a level which matches the pulse width when the pulse rate is high. Then, however, noise elimination performance drops in the case where the pulse rate is low. Accordingly, the present invention provides an arrangement such that the evaluation reference of the pulse width evaluation means 2 changes with the level of the pulse rate.

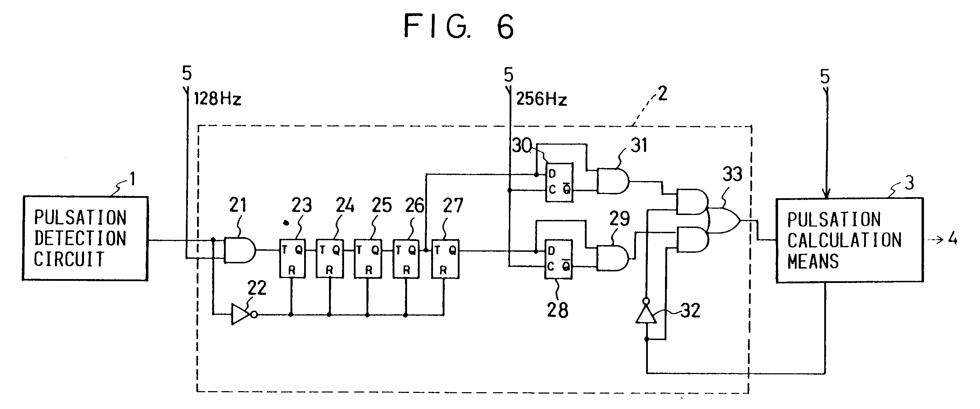

Next, a first specific embodiment of the present invention will be described with reference to Figure 6, which shows the circuitry of the pulse width evaluation means 2. The pulsation detection circuit 1 and the pulsation calculation means 3 are of known type and so their detailed circuitry is omitted. While the pulse signal output from the pulsation detection circuit 1 is at a level "1", a reference frequency from the reference signal generation circuit 5, such as 128 Hz, is output to the output of an AND gate 21. While the pulse signal is at the level "1" also, the re-set input of a binary counter consisting of T-type flip flops (hereinafter referred to as "TFF") 23 to 27 is released and the TFFs 23 to 27 count the 128 Hz output of the AND gate 21. When eight 128 Hz outputs of the AND gate 21 are counted, the Q output of the TFF 26 rises to "1" The Q output of the TFF 26 is connected to a differential circuit, consisting of a D-type flip flop (hereinafter referred to as "DFF") 30 and an AND gate 31, such that a pulse having a certain width is generated at the output of the AND gate 31 the instant that the Q output of the TFF 26 becomes "1". A further reference frequency, such as 256 Hz, from the reference signal generation circuit 5 is supplied to the clock terminal C of the DFF 30, whereby this pulse has a pulse width of 1.95 ms. The time till the generation of the pulse at the output of this differential circuit is about 62.5 ms from the output of the pulse signal from the pulsation detection circuit 1. The reason why this time is "about" is that the output of the pulse signal from the pulsation detection circuit 1 and the signal of the reference signal generation circuit 5 are not in synchronism with each other. Because they are asynchronous, the error of the pulse rate generated is about ±6 at a pulse rate of about 210. One method of reducing this error is to use a higher frequency than the 128 Hz supplied to the AND gate 21.

After the pulse is generated at the output of the AND gate 31 following the passage of about 62.5 ms, eight 128 Hz outputs of the AND gate 21 are further counted and then the Q output of the TFF 27 rises to "1". The Q output of the TFF 27 is connected to another differential circuit, consisting of a DFF 28 and an AND gate 29, such that a further pulse having a pulse width of 1.95 ms is generated at the output of the AND gate 29 the instant that the Q output of the TFF 27 becomes "1". The time till the generation of the further pulse at the Q output of the TFF 27 is about 125 ms after the output of the pulse signal from the pulsation detection circuit 1. The output of the AND gate 31 after the passage of about 62.5 ms and the output of the AND gate 29 after the passage of about 125 ms are supplied to a multiplexer consisting of an AND/OR gate 33 and an inverter 32. The multiplexer outputs one of the outputs of the AND gate 31 and 29 to the pulsation calculation means 3 in accordance with a control signal received from the pulsation calculation means 3. If the pulsation calculation means 3 is constituted so that the control signal becomes "1" when the previous calculation result of the pulsation calculation means is below a pulse rate of 100 and becomes "0" when the latter is above a pulse rate of 100, the pulsation calculation means 3 will make the pulsation calculation when there is an input of the pulse signal having a pulse width of at least about 125 ms when the pulse rate is below 100. And it will make the pulsation calculation when there is an input of the pulse signal having a pulse width of at least about 62.5 ms when the pulse rate is above 100.

With the pulse width evaluation means 2 disposed between the pulsation detection circuit 1 and the pulsation calculation means 3 as described above, the pulse rate can be counted when the pulse signal has a pulse width above a certain pre-determined width. As a result, noise having a small pulse width is neglected, and so the pulsation display 4 can be made stable.

Although two evaluation references for the pulse width of the pulse signal are provided in this embodiment, the number can easily be increased and noise elimination performance can be improved further by so doing.

When the pulse rate is high or when the pulse width of the pulse signal is relative small, there is a possibility that measurement might be impossible if a long evaluation reference is applied. However, this can be solved easily by selecting the values of the evaluation references and of the reference frequencies and does not at all constitute a drawback in the present invention. For example, if the pulse rate cannot be obtained easily, it is possible to employ an arrangement which automatically selects a minimum evaluation reference. In this type of pulsimeter, the pulse width evaluation means has storing means for storing a minimum evaluation reference and selecting means for selecting a respective evaluation reference from data stored in the storing means.

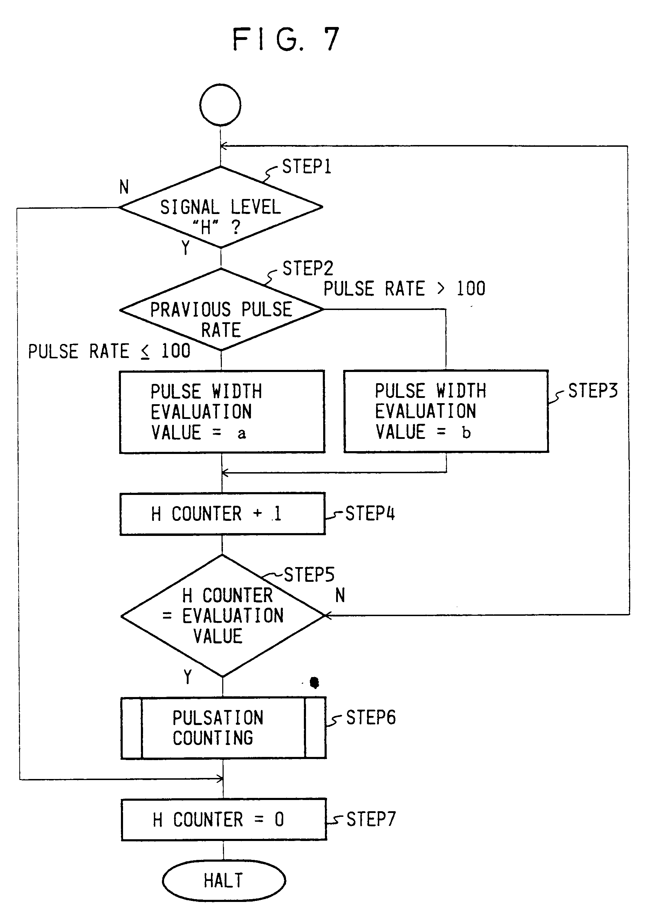

Next, another embodiment of the present invention and its operation will be explained. This embodiment represents the case where the pulse width evaluation means 2 and the pulsation calculation means 3 described above in relation to Figure 1 are accomplished by software in a micro-processor as illustrated in a flow chart shown in Figure 7.

With reference to Figure 7, an interrupt occurs at the rise of the pulse signal and the software is started. In the first step, STEP 1, the signal level of the pulse signal is checked and if it is "H", the process advances to STEP 2. In this STEP 2, whether or not the previous pulse rate displayed is above 100 is checked. If it is found to be less than or equal to 100 as the result of the check, the pulse width evaluation reference value is set to a. If it proves to be above 100, the pulse width evaluation reference value is set to b (STEP 3). In the next step, STEP 4, an H counter is incremented by +1. In the next step, STEP 5, a judgement is made as to whether or not the count value of the H counter is equal to the pre-determined pulse width evaluation reference value and, if it is, a pulsation counting sub-routine is effected in STEP 6 to count the pulse rate. If not, the process returns to the STEP 1 and repeats the loop including the STEPs 1 to 5 until the value of the H counter becomes equal to the pulse width evaluation reference value. In the meantime, if the signal level of the pulse signal falls to "L", the H counter is cleared in STEP 7 and the process shifts to HALT. As a result of the processing described above, the pulse rate calculation cannot be made unless the pulse signal has a certain pre-determined pulse width. Noise having a small pulse width can thus be neglected, and so the pulse rate display can be stabilised.

In accordance with the present invention described above, the pulse width evaluation means for evaluating the pulse signal output from the pulsation detection circuit can remove noise and transmit only the original pulse signal to the pulsation calculation means. Accordingly, the stability of the pulse rate display can be improved drastically.

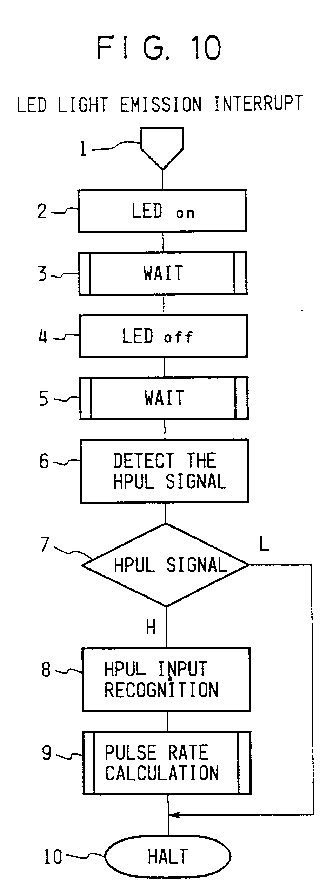

Figure 10 is a flow chart representing software for detecting the HPUL signal generated in the circuit, which is illustrated in Figure 8 and which has previously been described, and for programming the CPU 118 according to the present invention.

When a periodic (128 Hz in this embodiment) light emission interrupt is applied to the LED 111 to cause interrupted light emission, an LED-on command 2 and LED-off command 4 are executed sequentially. The LED-on time is a time where the change of the blood flow rate can be grasped as an electric signal through the light reception device by a WAIT routine 3. There is the possibility that a spike pulse due to the current change occurs during this LED-on time. Therefore, level detection of the HPUL signal is not made. A stand by is then provided by a WAIT routine 5 until the power source gets steady and the spike pulse disappears after LED-off. Then, the level of the HPUL signal b is detected (STEP 6). If the level of the HPUL signal b is "L" (STEP 7), the pulse signal is not judged as existing and the process proceeds to HALT 10. If the level of the HPUL signal b is "H", the pulse signal is accepted and HPUL input recognition processing (STEP 8) and pulse rate calculation processing (STEP 9) etc. are executed for confirming the pulse rate etc.

In the embodiment described above, level detection of the HPUL signal b is made whenever the LED 111 emits light but it is also possible to effect level detection in a shorter period, depending on the accuracy of the pulse rate detection, or to detect the periods other than the time from LED-on to LED-off and a subsequent specific period.

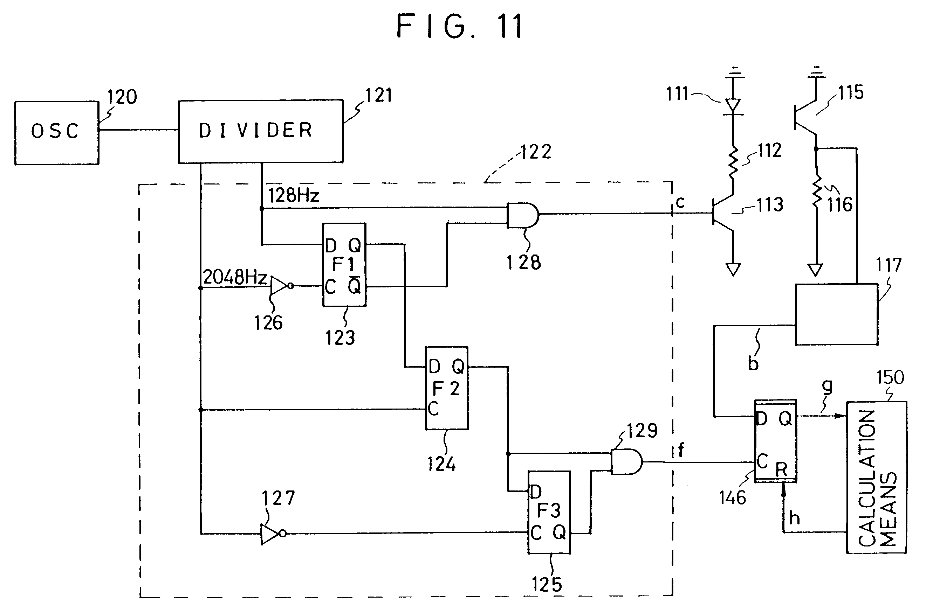

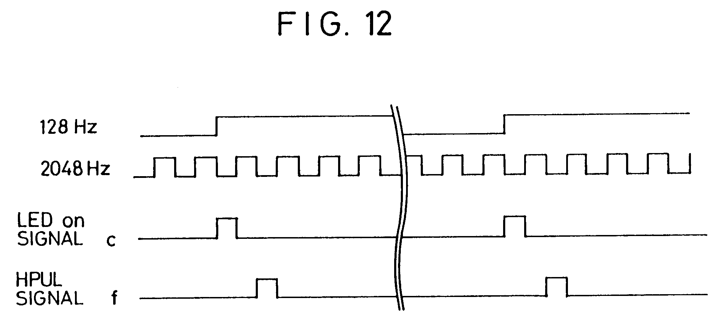

Next, a further modification of the circuit of Figure 8 according to the invention will be explained with reference to Figure 11, which is a schematic diagram of the circuit including pulse detection means 122. Assuming the LED light emission period is 128 Hz and the LED light emission time if 4096 Hz, for example, the pulse detection means 122 are arranged to receive 2048 Hz and 128 Hz signals, respectively, output from an oscillator OSC 120 by way of a frequency divider 121. The pulse detection means 122 outputs an LED light emission signal c having a pulse width of 4096 Hz in synchronism with the rise of the 128 Hz signal by means of a NOT circuit 126, a flip flop circuit 123 and a gate circuit 128. The pulse detection means 122 also outputs an HPUL detection signal f, which is delayed by 4096 Hz from the LED light emission signal c, by means of a NOT circuit 127, flip flop circuits 124 and 125, and a gate circuit 129. Figure 12 shows the relationship between the LED light emission signal c and the HPUL detection signal f.

The LED light emission signal c described above causes light emission of the LED 111 connected to the switching transistor 113 for controlling LED light emission. The HPUL detection signal f is input to the clock input of a half latch 126 for generating the HPUL signal with a delay of 4096 Hz. The half latch 126 employs the HPUL signal b output from the amplification circuit 117 as data, and latches the data in response to the clock signal input thereto with the delay of 4096 Hz from the LED light emission period. Thus, it becomes possible to output only the correct pulses as the pulse signal g to calculation means 150 consisting of a CPU or logic circuit, and to avoid taking any spike pulse generated during the LED light emission period as data. After outputting the pulse signal g, the half latch 146 receives a re-set signal h from the calculation means 150 or the like, and enters a stand by state to wait for the input of the next HPUL signal.

When the pulse signal is detected in periods other than the LED light emission period by use of means as described above, it becomes possible to prevent erroneous recognition of the spike pulses occurring at the time of application of the power source voltage as the pulse signal. Therefore, the correct pulsation can be detected and displayed stably without being affected by the life of the battery, the ambient temperature and the like.

## Claims

1. A photoelectric pulsation type pulsimeter comprising a pulsation detection circuit (1), which includes a photoelectric element for detecting blood flow rate and for generating a corresponding pulse signal, pulsation calculation means (3) for calculating a pulse rate in response to the pulse signal, reference signal generating means (5) for generating a reference signal and for outputting the reference signal to the pulsation calculation means (3), characterised in that the pulsimeter includes pulse width evaluation means (2) to which the reference signal is outputted, in that the pulse width evaluation means (2) evaluates the pulse signal output from the pulsation detection circuit (1) and in that the pulse width evaluation means (2) are arranged to change the evaluation reference according to a pulse rate previously calculated by the pulsation calculation means (3).

2. A pulsimeter as claimed in claim 1 characterised in that the pulse width evaluation means (2) are arranged to employ plural evaluation references.

3. A pulsimeter as claimed in claim 2 characterised in that the pulse width evaluation means (2) include storing means for storing evaluation references and selecting means for selecting a respective evaluation reference from the data of the storing means.

4. A pulsimeter as claimed in any preceding claim characterised in that the pulsation detection circuit (1) includes a light emission device (111).

5. A pulsimeter as claimed in claim 4 characterised in that the pulsation detection circuit (1) is arranged to detect the pulse signal in a period other than the turn on period of the light emission device (111).

6. A pulsimeter as claimed in claim 5 characterised in that the pulsation detection circuit (1) includes means (122) for masking the pulse signal in a specific period after turn on of the light emission device (111).

7. A pulsimeter as claimed in any preceding claim characterised by display means (4) for displaying the pulse rate calculated by the pulsation calculation means (3).

## Patentansprüche

1. Photoelektrischer Pulsmesser des Pulsationstyps, umfassend eine Pulsationserfassungsschaltung (1), welche ein photoelektrisches Element zum Erfassen einer Blutflußrate und zum Erzeugen eines entsprechenden Impulssignals umfaßt, ein Pulsationsberechnungsmittel (3) zum Berechnen einer Impulsrate in Antwort auf das Impulssignal, ein Referenzsignalerzeugungsmittel (5) zum Erzeugen eines Referenzsignals und zum Ausgeben des Referenzsignals zu dem Pulsationsberechnungsmittel (3),
dadurch gekennzeichnet,
daß der Pulsmesser ein Impulsbreitenauswertemittel (2) umfaßt, zu welchem das Referenzsignal ausgegeben wird, daß das Impulsbreitenauswertemittel (2) das von der Pulsationserfassungsschaltung (1) ausgegebene Impulssignal auswertet, und daß das Impulsbreitenauswertemittel (2) dazu eingerichtet ist, die Auswertereferenz gemäß einer durch das Pulsationsberechnungsmittel (3) früher berechneten Impulsrate zu ändern.

2. Pulsmesser nach Anspruch 1,
dadurch gekennzeichnet,
daß das Impulsbreitenauswertemittel (2) dazu eingerichtet ist, mehrere Auswertereferenzen zu verwenden.

3. Pulsmesser nach Anspruch 2,
dadurch gekennzeichnet,
daß das Impulsbreitenauswertemittel (2) Speichermittel umfaßt zum Speichern von Auswertereferenzen und Auswahlmittel zum Auswählen einer jeweiligen Auswertereferenz aus den Daten der Speichermittel.

4. Pulsmesser nach einem der vorhergehenden Ansprüche,
dadurch gekennzeichnet,
daß die Pulsationserfassungsschaltung (1) eine Lichtemissionsvorrichtung (111) umfaßt.

5. Pulsmesser nach Anspruch 4,
dadurch gekennzeichnet,
daß die Pulsationserfassungsschaltung (1) dazu eingerichtet ist, das Impulssignal in einer Periode zu erfassen, welche sich von der Einschaltperiode der Lichtemissions-Vorrichtung (111) unterscheidet.

6. Pulsmesser nach Anspruch 5,
dadurch gekennzeichnet,
daß die Pulsationserfassungsschaltung (1) Mittel (122) umfaßt zum Ausblenden des Impulssignals in einer spezifischen Periode nach dem Anschalten der Lichtemissionsvorrichtung (111).

7. Pulsmesser nach einem der Vorhergehenden Ansprüche,
gekennzeichnet durch
Anzeigemittel (4) zum Anzeigen der durch die Pulsationsberechnungsmittel (3) berechneten Impulsrate.

## Revendications

1. Sphygmomètre du type à pulsation photoélectrique comprenant un circuit (1) de détection de pulsation qui comporte un élément photoélectrique pour détecter le débit sanguin et pour produire un signal impulsionnel correspondant, des moyens de calcul de pulsation (3) pour calculer une fréquence de pouls en réponse au signal impulsionnel, des moyens générateurs de signal de référence (5) pour produire un signal de référence et pour fournir en sortie le signal de référence aux moyens de calcul de pulsation (3), caractérisé en ce que le sphygmomètre comporte des moyens d'évaluation de la largeur d'impulsion (2) auxquels est fourni en sortie le signal de référence, en ce que les moyens d'évaluation de la largeur d'impulsion (2) évaluent la sortie de signal impulsionnel du circuit de détection de pulsation (1) et en ce que les moyens d'évaluation de la largeur d'impulsion (2) sont conçus pour modifier la référence d'évaluation en fonction de la fréquence du pouls préalablement calculée par les moyens de calcul de pulsation (3).

2. Sphygmomètre selon la revendication 1, caractérisé en ce que les moyens d'évaluation de la largeur d'impulsion (2) sont conçus pour utiliser plusieurs références d'évaluation.

3. Sphygmomètre selon la revendication 2, caractérisé en ce que les moyens d'évaluation de la largeur d'impulsion (2) comprennent des moyens de stockage pour stocker des références d'évaluation et des moyens de sélection pour sélectionner une référence d'évaluation correspondante parmi les données des moyens de stockage.

4. Sphygmomètre selon l'une quelconque des revendications précédentes, caractérisé en ce que le circuit de détection de pulsation (1) comporte un dispositif d'émission lumineuse (111).

5. Sphygmomètre selon la revendication 4, caractérisé en ce que le circuit de détection de pulsation (1) est conçu de manière à détecter le signal impulsionnel au cours d'une période différente de la période d'activation du dispositif d'émission lumineuse (111).

6. Sphygmomètre selon la revendication 5, caractérisé en ce que le circuit de détection de pulsation (1) comporte des moyens (122) pour masquer le signal impulsionnel au cours d'une période particulière après l'activation du dispositif d'émission lumineuse (111).

7. Sphygmomètre selon l'une quelconque des revendications précédentes, caractérisé par des moyens d'affichage (4) pour afficher la fréquence de pouls calculée par les moyens de calcul de pulsation (3).
